(19) Europäisches Patentamt
European Patent Office
Office européen des brevets

(11) Publication number: **0 206 765 B1**

(12) **EUROPEAN PATENT SPECIFICATION**

(45) Date of publication of patent specification: **06.03.91**

(51) Int. Cl.5: **C07C 33/048**, C07C 39/21, C07H 15/203, C07C 43/215

(21) Application number: **86304715.5**

(22) Date of filing: **19.06.86**

The file contains technical information submitted after the application was filed and not included in this specification

# (54) Process for preparation of rooperol, hypoxoside and derivatives thereof.

(30) Priority: **19.06.85 ZA 854640**

(43) Date of publication of application: **30.12.86 Bulletin 86/52**

(45) Publication of the grant of the patent: **06.03.91 Bulletin 91/10**

(84) Designated Contracting States: **AT BE CH DE FR GB IT LI LU NL SE**

(56) References cited:
**EP-A- 0 130 829**
**GB-A- 2 120 650**

**T.W. GREENE "Protective groups in organic synthesis", 1981, pages 100, 101, John Wiley & Sons, New York. Chichester. Brisbane. Toronto**

**CHEMICAL ABSTRACTS, vol. 78, no. 5, 05 Feb 1973, Columbus, OH (US); E.J.COREY et al.: "Synthetic method for conversion of formyl groups into ethynyl groups", p. 456, no. 29167f**

**TETRAHEDRON LETTERS, vol. 25, no. 13, 1984, pages 1379-1382, GB; S. HASHIMOTO et al.: "Glycosylation using glucopyranosyl fluorides and silicon-based catalysts. Solvent dependency of the stereoselection"**

(73) Proprietor: **ROOPEROL (NA) NV**
**C.E.B. Hellmundweg 11**
**Kralendijk BonaireNetherlands Antilles(NL)**

(72) Inventor: **Wenteler, George L.**
**73, Kafue Street**
**Lynwood Glen Pretoria(ZA)**
Inventor: **Pegel, Karl H.**
**135 Manor Drive**
**Durban Natal(ZA)**
Inventor: **Drewes, Siegfried**
**76 Blackburrow Road**
**Pietermaritzburg(ZA)**

(74) Representative: **Holmes, Michael John et al**
**Frank B. Dehn & Co. European Patent Attorneys Imperial House 15-19 Kingsway**
**London, WC2B 6UZ,(GB)**

**EP 0 206 765 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to the preparation of a glucoside named Hypoxoside, namely 1,5-bis[3'4', 3",4"-tetrahydroxyphenyl]pent-4-en-1-ynyl-4-4"-di[β-D-glucopyranoside], its aglycone Rooperol, namely 1,5-bis-[3'4',3",4"-tetrahydroxyphenyl]pent-4-en-1-yne and some of its analogues and derivatives. The synthesis requires the use of tert -butyl dimethylsilyl ethers or other suitable protecting groups. The glycosylation of rooperol and its derivatives is also described.

The isolation of E-1,5-bis(3',4'-dihydroxyphenyl)-pent-4-en-1-yne-4',4'-di-β-D-glucopyranoside, Hypoxoside, from Hypoxis obtusa and Hypoxis rooperi has been described by Marini-Bettolo et al (Tetrahedron 1982, 38 , pp 1683-1687) and Drewes et al (Phytochemistry, 1984, 23 , pp 1315-1316). The use of Hypoxoside, Rooperol and related pent-4-en-1-yne derivatives in anticancer compositions has been disclosed (European Patent Application No. 843044983). The preparation of Rooperol by the hydrolysis of Hypoxoside in water at pH 6.3 with β-glucosidase at preferably 37°C, the preparation of the tetra-acetate from Rooperol by acylation with ($Ac_2O/C_5H_5N$), and also the preparation of tetramethoxyrooperol from Rooperol by methylation with diazomethane has been disclosed (Marini-Bettolo et al, Drewes et al, references as above). The synthesis of the tetramethoxy derivative of Rooperol is achieved by the conversion of 3,4-dimethoxybenzaldehyde to the alkynide anion of 3,4-dimethoxyethynylbenzene (3 steps) and subsequent coupling to 3,4-dimethoxycinnamyl chloride obtained from 3-(3',4'-dimethoxyphenyl) prop-2-enoic acid (3 steps) via copper catalysis. Similar synthetic sequences using phenyl rings with no substituents or precursors in which one or both phenyl rings carry methoxy- or methylenedioxy substituents, have been described (Drewes et al, reference as above). Although a variety of protecting groups have been used, neither the removal of these protecting groups to yield Rooperol, nor the glucosidation of Rooperol to yield Hypoxoside, have been described. It is the object of this invention to provide a process for the synthesis of Rooperol and related derivatives as well as Hypoxoside and related glucosides.

The overall process according to the invention comprises the synthesis of a protected hydroxycinnamyl aldehyde or a corresponding allylic haloalkane and the reaction thereof with an alkylating agent such as phenylacetylide anion or a protected hydroxyphenylacetylide anion to form a variety of substituted pent-4-en-1-yne compounds followed by deprotection to yield the hydroxy derivatives.

According to one aspect the present invention provides a process for preparing compounds of formula (I)

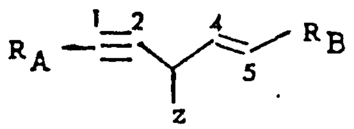

(I)

(wherein,
$R_A$ represents an unsubstituted phenyl group or a phenyl group substituted by up to 5 groups selected from hydroxy, alkoxy, alkylenedioxy, halide and acyloxy groups;
$R_B$ represents an unsubstituted phenyl group or a phenyl group substituted by up to 5 groups selected from hydroxy, alkoxy, alkylenedioxy, halide and acyloxy groups;
at least one of the groups $R_A$ and $R_B$ carrying at least one hydroxy substituent; and
Z represents a hydrogen or hydroxy group);
which comprises subjecting a compound of formula (II)

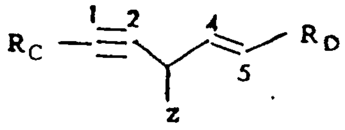

(II)

(wherein,
$R_C$ represents an unsubstituted phenyl group or a phenyl group substituted by up to 5 groups selected from tert-butyl-dimethylsilyloxy, alkoxy, alkylenedioxy, halide and acyloxy groups;
$R_D$ represents an unsubstituted phenyl group or a phenyl group substituted by up to 5 groups selected from

tert-butyl-dimethylsilyloxy, alkoxy, alkylenedioxy, halide and acyloxy groups;
at least one of the groups $R_C$ and $R_D$ carrying at least one trialkylsilyloxy substituent; and
Z is as defined above),
to alkylammonium fluoride or acid mediated hydrolysis.

The process according to the invention is preferably a process as hereinbefore described for preparing compounds of formula (I)

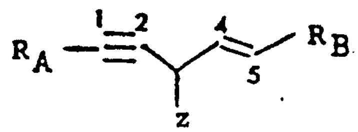

(I)

(wherein,
$R_A$, $R_B$ and Z are as hereinbefore defined)
which comprises treating a compound of formula (III)

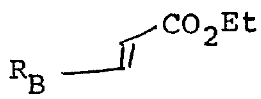

(III)

(wherein $R_B$ is as hereinbefore defined) with tert-butyldimethyl silyl chloride in the presence of a base, reducing the resultant compound of formula (IV)

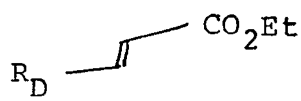

(IV)

(wherein $R_D$ is as hereinbefore defined) to the aldehyde or alcohol, optionally converting the resultant alcohol to an allylic halide of formula (V)

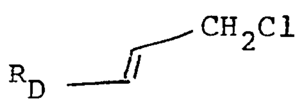

(V)

(wherein $R_D$ is as defined above) reacting said allylic halide or said aldehyde with a compound of formula (VI)

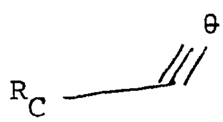

(VI)

(wherein $R_C$ is as hereinbefore defined) to form a compound of formula (II) (as hereinbefore defined) and subjecting said compound of formula (II) to alkylammonium fluoride or acid mediated hydrolysis.

Further, according to the invention a protected or unprotected compound may be glycosylated with a halosugar to yield a glycoside.

Thus, according to a further aspect the present invention provides a process for preparing compounds of formula (IX)

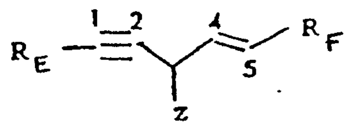

(IX)

(wherein,

$R_E$ represents an unsubstituted phenyl group or a phenyl group substituted by up to 5 groups selected from glucosyloxy, hydroxy, alkoxy, alkylenedioxy, halide and acyloxy groups;

$R_F$ represents an unsubstituted phenyl group or a phenyl group substituted by up to 5 groups selected from glucosyloxy, hydroxy, alkoxy, alkylenedioxy, halide and acyloxy groups;

at least one of the groups $R_E$ and $R_F$ carrying at least one glucosyloxy group; and

Z is as hereinbefore defined);

which comprises the glucosidation of a compound of Formula (I) or of Formula (II) in a consecutive step to its prepartion by the process as hereinbefore described wherein the Formulae (I) and (II) are as hereinbefore defined.

In a preferred form of the invention the phenolic hydroxy groups in caffeic acid ester are protected by their conversion to silyl ethers by using tert butyldimethylsilyl chloride and a base such as imidazole. The silyloxy ester is reduced to the aldehyde by means of DIBAH and to the corresponding alcohoi with an excess of DIBAH or $LiAlH_4$.

Direct reduction of silyloxycaffeic acid to the alcohol may be achieved by its treatment with $B_2H_6$. The conversion of the alcohol to the corresponding halide proceeds smoothly by conventional methods. The silyl ether of 3,4-dihydroxyphenylacetylene may be prepared as shown in SCHEME 2 by:

(i) treating 3,4-dihydroxybenzaldehyde with tert -butyldimethylsilyl chloride and imidazole as a base;

(ii) conversion of a carbonyl group to the corresponding dibromide by the method of E J Corey (Tetrahedron Lett. 1972, pp 3769-3772) followed by dehydrohalogenation with two equivalents of butyl lithium;

(iii) alkylation with different $\alpha,\beta$- unsaturated aldehydes or allylic haloalkanes yields the corresponding substituted pent-4-en-1-yne derivatives;

(iv) deprotection of the silyl ethers with a tetraalkyl ammonium fluoride, or the like, e.g. tetraethyl ammonium fluoride, yields the corresponding alcohols as shown in SCHEME 2.

By treating 3-(3',4'-tert -butyldimethylsiloxyphenyl) prop-2-enal or 3-(3',4'-tert -butyldimethylsiloxyphenyl)-1-haloprop-2-ene with phenylacetylene magnesium bromide

1-phenyl-5-(3',4'-tert -butyldimethylsiloxyphenyl)pent-4-en-1-yn-3-ol or

1-phenyl-5-(3',4'-tert -butyldimethylsiloxyphenyl)pent-4-en-1-yne were obtained. Deprotection of the siloxy products with tetraalkyl ammonium fluoride or the like yields the corresponding hydroxy compounds (SCHEMES 1 and 2).

Glycosylation of the phenolic hydroxy groups by the classical Koenigs Knorr rection (Koenigs et al, Ber 1901, 34 , pp 957-981) or modified procedures (H Paulsen: Angew. Chem. Int. Engl. Ed. 1982, 21 , 155 and Angew. Chem. 1982, 94 , pp. 184-201; S R Pougny et al, J. Am. Chem. Soc. 1977, 99 , pp 6762-6763; K C Nicolaou et al, J. Am. Chem. Soc. 1983, 105 , pp 2430-2434), yielded the corresponding glycosides. The preferred glycoside group is the $\beta$-D-glucoside substituent. Treatment of 2,3,4,6-tetra-O-benzyl-$\alpha$-D-glucopyranosyl fluoride (S Hasimoto et al, Tetrahedron Lett. 1984, pp 1379-1382) with the silyl ethers yielded the products as shown in SCHEME 2.

Scheme 1

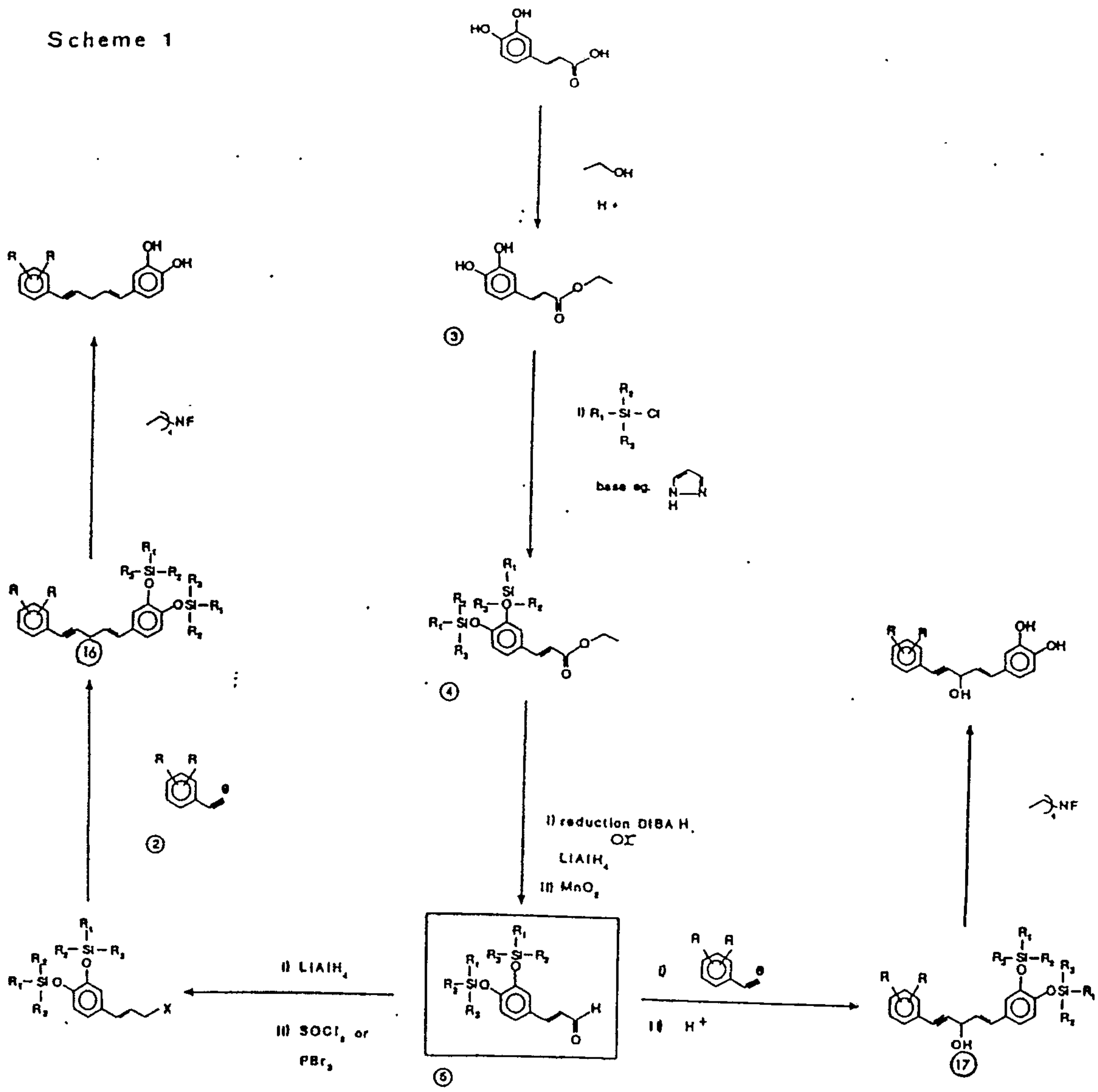


Where R = hydrogen, OMe, Halide, -O-CH$_2$-O, acyl

$R_1 = R_2 = CH_3$

$R_3$ =

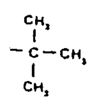

X = Halogen

Scheme 2

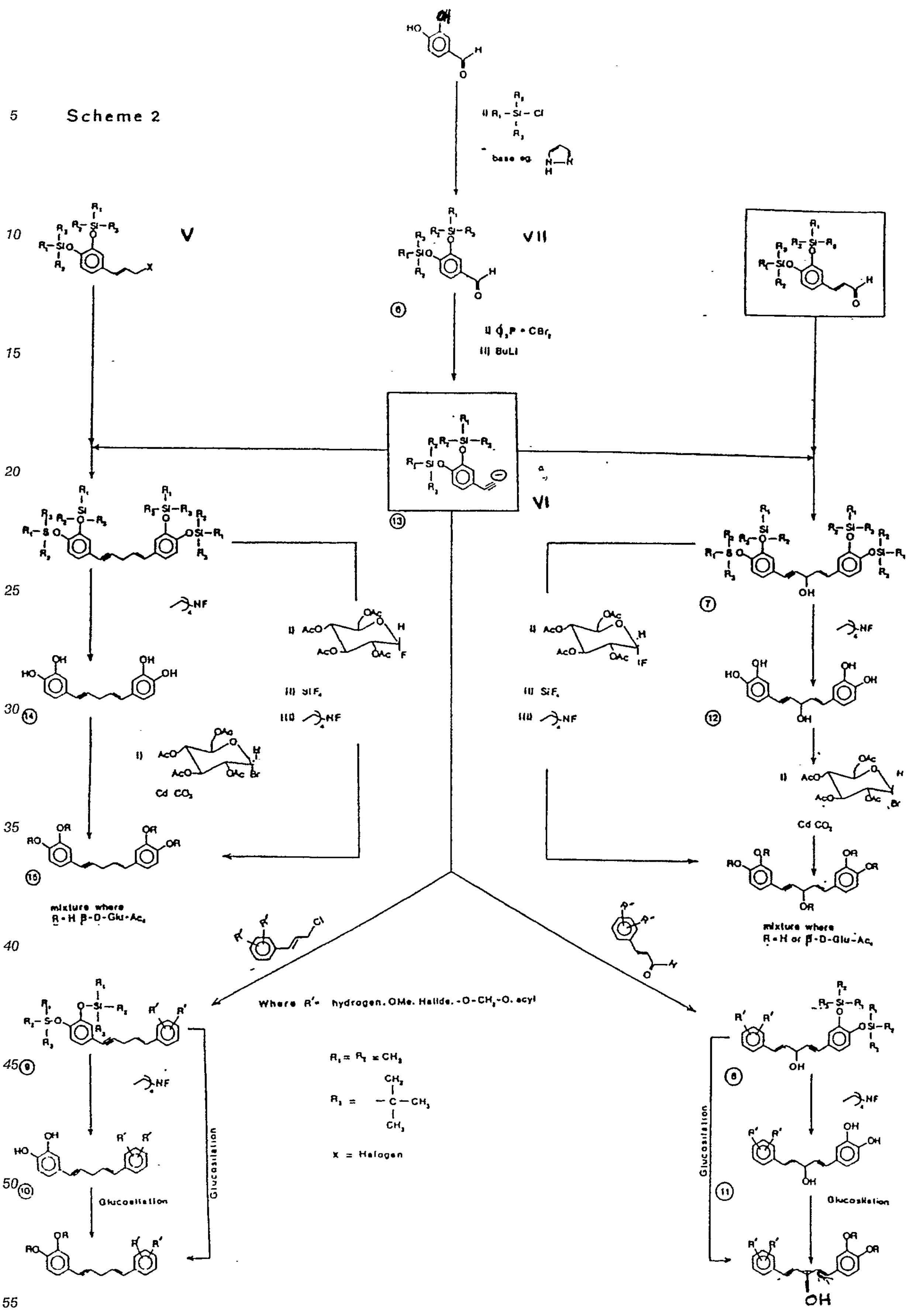
i) R1-Si-Cl
base eg.
V
VII
(6)
i) ϕ3P + CBr4
ii) BuLi
VI
(13)
(7)
(14)
(12)
(15)
i) SiF4
iii) NF
i) CdCO3
mixture where R = H β-D-Glu-Ac4
mixture where R = H or β-D-Glu-Ac4
Where R' = hydrogen, OMe, Halide, -O-CH2-O- acyl
R1 = R2 = CH3
X = Halogen
(9)
(8)
(10)
(11)
Glucosilation

PROCEDURES:

I Synthesis of the ethyl ester of caffeic acid (3 in SCHEME 1)

Method:

A solution of caffeic acid (10.0 g, 0.055 mol) in a mixture of benzene (300 ml), ethanol (80 ml) and $H_2SO_4$(conc. 5 ml) is refluxed for 34 hours. The cooled dark green reaction mixture is neutralized with a saturated aqueous $NaHCO_3$ solution. The aqueous layer is then extracted with ether (3 x 15 ml), dried ($MgSO_4$) and the combined organic extracts concentrated to yield a brown crystalline product (9.0 g, 78%). Recrystallization from ethyl acetate yielded the ethyl ester of caffeic acid as a light brown crystalline product (5.98 g, 52%) mp 143-146 °C;
$\delta_H$(acetone; 80 MHz; TMS): 1.27(3 H, t, J = 7.1 Hz, $CH_2CH_3$);
4.2(2 H, t, J = 7.1 Hz, $CH_2CH_3$), 6.27(1 H, d, J = 15.9 Hz, H-2)
6.82-7.18(3 H, m, Ar H ), and 7.55(1 H, d, J = 15.9 Hz, H-3)

II Synthesis of 3-[3',4'-di(tert-butyldimethylsiloxy)phenyl] prop-2-en-1-ol :

Method:

DIBAH (175 mg, 0.137 mmol) in toluene (3.3 ml) is added to a solution of the disilyl ether of the ester (4) (500 mg, 0.114 mmol) in toluene (1.5 ml) at -78 °C over a period of 1 hour. The reaction mixture is then allowed to reach room temperature when it is quenched with saturated aqueous $NH_4Cl$ (10 ml) and extracted with ethyl acetate (3 x 10 ml). The combined extracts are dried ($MgSO_4$) and concentrated to provide a light yellow oil of 3-[3',4'-di( *tert* -butyldimethylsiloxy)phenyl]prop-2-en1-ol (350 mg, 78%); $\delta_H$-($CDCl_3$; 80 MHz; TMS): 0.25(12 H, s, Si-$CH_3$), 0.9(18 H, s, Si+$CH_3$), 1.22-1.28(2 H, m, $CH_2$), 4.22(1 H, d, J = 5.1 Hz, H-2), 6.20(1 H, d, J = 5.1 Hz, H-3) and 6.69-6.85(3 H, m, Ar H )

III Synthesis of 3-[3',4'-di(tert-butyldimethylsiloxy)phenyl] prop-2-enal :

Method :

$MnO_2$ (2.95 g; 0.034 mol) is added to a solution of 3-[(3',4'-*tert* -butyldimethylsiloxy)phenyl]prop-2-en-1-ol (250 mg, 0.0064 mol) in $CH_2Cl_2$ (10 ml). The reaction mixture is stirred for 24 hours and a light yellow oil of 5 (220 mg, 76%) is obtained after filtration and evaporation of the solvent under reduced pressure.
$\delta_H$($CDCl_3$; 80 MHz, TMS): 0.23(12 H, s, -Si-$CH_3$),
0.9(18 H, s, Si+$CH_3$), 6.52(1 H, dd, J = 7.6 Hz and J = 16 Hz, H-2),
6.99-7.11(3 H, m, Ar H ), 7.36(1 H, d, J = 16 Hz, H-3) and 9.64(1 H, d, J = 7.6 Hz, CHO).

IV Synthesis of 3,4-di(tert-butyldimethylsiloxy)-2',2'-dibromostyrene :

Method :

A suspension of activated zinc (1.90 g, 0.029 mol, 2 eqv), triphenylphosphine (7.60 g, 0.0029 mol, 2 eqv) and carbon tetrabromide (9.60 g, 0.029 mol, 2 eqv) in dry $CH_2Cl_2$ (20 ml) is stirred for 30 hours under $N_2$. To this Wittig reagent is added
3,4-di( *tert* -butyldimethylsiloxy)benzaldehyde (5.50 g, 0.015 mol) and the mixture is stirred for 3 hours at

room temperature before it is quenched with pentane (50 ml) and filtered. The residue is washed with $CH_2Cl_2$ (30 ml) and the organic solvents are evaporated to provide the dibromide as a yellow oil (5.08 g, 66%). $\delta_H$($CDCl_3$; 80 MHz, TMS):
0.25(12 H, s, -Si-C $\underline{H}_3$), 0.90(18 H, s, Si+C $\underline{H}_3$) and 6.82(1 H, s, C $\underline{H}$ ) and 7.20-7.33(3 H, m, Ar $\underline{H}$ )

V Synthesis of 3',4'-di(tert-butyldimethylsiloxy)phenylethyne :

Method :

To a solution of 3,4-di( *tert* -butyldimethylsiloxy)-2',2'-dibromostyrene (3.00 g, 0.006 mol) in dry tetrahydrofuran (10 ml) is added BuLi (2 eqv) at -78 °C over a period of 1 hour. The reaction mixture is allowed to reach room temperature when it is quenched with saturated aq. $NaHCO_3$ (20 ml), extracted with ethyl acetate (3 x 20 ml) and the combined extracts are dried ($MgSO_4$) before the solvent was evaporated under reduced pressure to provide the corresponding acetylenic product as a dark brown oil (1.8 g, 86%).$\delta_H$($CDCl_3$; 80 MHz, TMS):
0.25(12 H, s, -Si-C $\underline{H}_3$), 0.9(18 H, s, Si+C $\underline{H}_3$), 2.96(1 H, s, C $\underline{H}$ ), and 6.69-7.26(3 H, m, Ar $\underline{H}$ ).

General Procedures :-

VI Protection of the aromatic OH groups as tert-butyldimethylsilyl ethers

A solution of the phenolic compound (1 eqv), *tert* -butyldimethylchlorosilane (1.1 eqv per OH group), and imidazole (1.2 eqv) in DMF is stirred for 24 hours at 23°C. The solution is quenched with a saturated aqueous solution of $NaHCO_3$ and extracted with ether. The combined extracts are washed with water, dried ($MgSO_4$), and the solvent evaporated to provide a crude product which is purified by suitable methods. As an example compounds 4 and 6,as in SCHEME 1 and 2,. have been prepared by the above methods.

Compound 4

$_H$($CDCl_3$; 80 MHz, TMS): 0.25(12 H, s, -Si-$CH_3$),
0.90(18 H, s, Si+C $\underline{H}_3$), 1.33(3 H, t, J = 7.08 Hz, $CH_2$C $\underline{H}_3$),
4.25(2 H, q, J = 7.08 Hz, C $\underline{H}_2$-$CH_3$), 6.22(l H, d, J = 15.8 Hz, H-2);
6.75-7.06(3 H, m, Ar $\underline{H}$ ) and 7.57(l H, d, J = 15.8 Hz, H-3).

Compound 6

H($CDCl_3$; 80 MHz, TMS): 0.25(12 H, s, Si-C $H_3$),
0.9(18 Ht s, Si+C $\underline{H}_3$), 6.75-7.43(3 H, m, Ar $\underline{H}$ ) and 9.80(1 H, S, C $\underline{H}$ O)

VII Alkylatlon of alkylhalides with phenylacetylide magnesium bromide 2

Bromoethane (1 mol equiv) is added dropwise to a stirred mixture of magnesium metal (1.05 mol equiv) and tetrahydrofuran under anhydrous conditions and a $N_2$ atmosphere. Once the exothermic reaction has subsided, the reaction mixture is refluxed for 10 minutes, cooled to 20 °C and the alkyne (as in SCHEMES 1 and 2) (1.05 mol equiv) dissolved in THF is added dropwise. Ethane is evolved during the formation of the phenylacetylide magnesium bromide. The mixture is then refluxed for 45 minutes and cooled to 20 ° C.

Dry cuprous chloride is added to the phenylacetylide magnesium bromide containing reaction mixture which is then stirred for 15 minutes before the alkyl halide (as in SCHEME 1 and 2) (1.05 mol eqv) dissolved in THF is added dropwise. The green suspension is refluxed for 45 minutes before an aqueous solution of ammonium chloride and KCN is added followed by ether extraction and work-up to yield the corresponding pent-4-ene-1-yn products. As example compound 16 (R = H), as in SCHEME 1, was

synthesised by the same method.
$_H$(DCl$_3$; 80 MHz, TMS): 0.23(12 H, s, -SiCH$_3$), 0.9(18 H, s, Si+CH$_3$), 3.35(2 H, d, J = 5 Hz, H-2,3), 6.0(1 H, dt, J = 5 Hz and J = 16 Hz, H-4), 6.83(1 H, d, J = 15 Hz, H-5) and 0.9-7.6(8 H, m, ArH).

VIII Alkylation of alkanals with phenylacetylene magnesium bromide (2)

The phenylacetylide magnesium bromide containing reaction mixture as prepared by method VII is cooled to 0° C and the alkanal (as in SCHEME 2) dissolved in THF is added dropwise maintaining the temperature between 0-5° C. After all the alkanal has been added the reaction mixture is stirred for a further 2.5 hours at 25° C and then poured into a saturated aqueous solution of $NH_4Cl$ followed by ether extraction and work-up to yield the corresponding pent-4-ene-1-yn-3-ol products. As example compound 17 was synthesised by this method $_H$(CDCl$_3$; 80 MHz, TMS):
0.25(12 H, s, -SiCH$_3$), 0.9(18 H, s, Si+CH$_3$), 5.19(1 H, d, J = 5.9 Hz, H-3), 6.24(1 H, dd, J = 5.9 Hz and J = 15 Hz), 6.8(1 H, d, J = 15 Hz, H-5) and 7.2-7.6(8 H, m, ArH).

IX Alkylation of alkyl halides with Lithium (3,4-di(tert-butyldimethylsiloxyphenyl)acetylide(13)

A mixture of cuprous chloride and 13 (1 mol eqv), as prepared by procedure V, in dry tetrahydrofuran is stirred for 15 minutes before the alkyl halide (as in SCHEME 2) (1.1 mol eqv) dissolved in dry THF is added dropwise. The green suspension is refluxed for 45 minutes before an aqueous solution of ammonium chloride and KCN is added followed by extraction with ethyl acetate and work-up to yield the corresponding silyl ether pent-4-ene-1-yne products. Deprotection of these provide, for example, compound 14 as described under Procedure XI.

X Alkylation of alkanals with Lithium 3,4-di(-tert-butyldimethylsiloxyphenyl)acetylide(13)

A THF solution of the phenylacetylide 13 as prepared by procedure V is cooled to 0° C and the alkanal in dry THF is added dropwise maintaining the temperature between 0-5° C. After all alkanal has been added the mixture is stirred for a further 2.5 hours at 25° C and then poured into a saturated aqueous solution of $NH_4Cl$ followed by extraction with ether and work-up to yield the corresponding pent-4-ene-1-yn-3-ol products. As example compounds 7 and 8, as in SCHEME 2, have been synthesised by this method.

Compound 7

$_H$(CDCl$_3$; 80 MHz, TMS): 0.25(24 H, s, Si-CH$_3$),
0.9(36 H, s, Si+CH$_3$), 5.18(1 H, d, J = 5.9 Hz, H-3),
6.24(1 H, dd, J = 5.9 Hz, J = 15 Hz, H-4), and
6.71-7.11(7 H, m, ArH, and H-5).

Compound 8

$_H$(CD$_3$COCD$_3$; 80 MHz, TM5): 0.25(12 H, s, Si-CH$_3$,
0.9(18 H, s, Si+CH$_3$), 5.19(1 H, d, J = 5.9 Hz, H-3),
6.24(1H, dd, J = 5.9 Hz and J = 15 Hz, H-4),
6.83(1 H, d, J = 15 Hz, H-5) and 7.2-7.6(8 H, m, ArH)

XI Deprotection of the silyl ether products

To a solution of the silyl ether (1 eqv) in dry THF is added tetraethyl ammonium fluoride (1.2 eqv per silyl ether group) in dry THF over a period of 15 minutes and the resulting mixture is stirred for an additional 3 hours at 23° C. The cooled reaction mixture is diluted with water and extracted several times with ethyl acetate. The combined extracts are dried ($MgSO_4$) and evaporated under reduced pressure. The

residue is purified according to standard procedure to provide the free phenolic products. As example compounds 11 and 14, as in SCHEME 2, have been synthesised by this method.

Compound 11 R' = H

$_{H}$($CDCl_3$; 80 MHz, TMS): 5.21(1 H, d, J = 5.9 Hz, H-3),
6.24(1 H, dd, J = 5.9 Hz and J = 16 Hz, H-4),
6.64(1H, d, J = 15 Hz, H-5) and 7.06-7.47(8 H, m, Ar H ).

Compound 14 (Rooperol)

$_{H}$($CD_3OD$; 80 MHz, TMS): 3.35(2 H, d, J = 5 Hz, H-2,3),
6.00(1 H, dt, J = 5 Hz and J = 16 Hz, H-4), 6.83(1 H, d, J = 15 Hz, H-5)
and 7.2-7.6(6 H, m, Ar H ).

XII Glucosidation of the silyl ethers :

To a solution of the 2,3,4,6-tetra -O-benzyl-αD-glucopyranosyl fluoride (2.1eqv) and the silyl ether (1 eqv) in acetonitrile is added a solution of tetrafluorosilane (0.25 eqv) in acetonitrile at 0 °C. The resulting mixture is stirred for 4 hours at 0 °C and then poured into a solution of potassium fluoride (5 g) and 0.1 M phosphate buffer (pH 7.4). The ethyl acetate extract of this reaction mixture is washed with an aqueous solution of $NaHCO_3$ and dried ($MgSO_4$) before solvent evaporation. The residue is chromatographed on silica gel to yield the different anomeric glucosides as shown in SCHEME 2.

XIII Glucosidation of the phenols :

Classical Koenigs-Knorr synthesis using $CdCO_3$ (or $Ag_2CO_3$• $Ag_2O$, HgCN) as catalyst and acid scavenger and
2,3,4,6-tetra-O-acetyl-α-D-glucopyranosyl bromide was used. Standard workup procedure followed to yield, after colum chromatography, the different anomeric glucosides as in SCHEME 2.

XIV Hydrolysis of tetraacetate or tertabenzoate Derivatives :

Methanolic KOH (4%) was added to a stirred solution of the tetraacetate or tetrabenzoate in $CHCl_3$ and the solution heated to just below the boiling point for 30 minutes. The resultant mixture was allowed to stand for 3 hours before it is again heated and stirred for 30 minutes. This procedure was repeated until t.l.c. checks showed that hydrolysis was complete. The reaction was worked up by standard procedures to yield a mixture of anomeric glucosides. As example compound 15 as in SCHEME 2 has been synthesised by this method.

Compound 15 (2x meta R = H, 2x para R = β-D-glucosyl)

$_{H}$($CD_3OD$; 80 MHz, TMS): 3.10-4.05(14 H, m, C $H_2$ and 12 glucosylprotons), 4.75(2 H, m, O H ), 6.06(1 H, dt, J = 15.0 and J= 50 Hz, H-4), 6.53(1 H, d, J = 15 Hz, H-5), 6.65-6.90(4 H, m, Ar H ) and 7.07(2 H, d, Ar H ).

## Claims

**1.** A process for preparing compounds of formula (I)

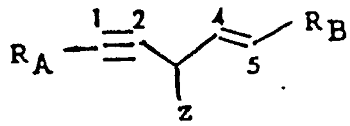

(I)

(wherein,
$R_A$ represents an unsubstituted phenyl group or a phenyl group substituted by up to 5 groups selected from hydroxy, alkoxy, alkylenedioxy, halide and acyloxy groups;
$R_B$ represents an unsubstituted phenyl group or a phenyl group substituted by up to 5 groups selected from hydroxy, alkoxy, alkylenedioxy, halide and acyloxy groups;
at least one of the groups $R_A$ and $R_B$ carrying at least one hydroxy substituent; and
Z represents a hydrogen or hydroxy group);
which comprises subjecting a compound of formula (II)

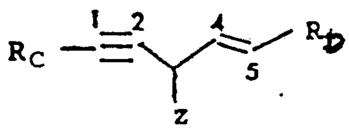

(II)

(wherein,
$R_C$ represents an unsubstituted phenyl group or a phenyl group substituted by up to 5 groups selected from tert-butyl-dimethylsilyloxy, alkoxy, alkylenedioxy, halide and acyloxy groups;
$R_D$ represents an unsubstituted phenyl group or a phenyl group substituted by up to 5 groups selected from tert-butyl-dimethylsilyloxy, alkoxy, alkylenedioxy, halide and acyloxy groups;
at least one of the groups $R_C$ and $R_D$ carrying at least one tert-butyldimethylsilyloxy substituent; and
Z is as defined above),
to alkylammonium fluoride or acid mediated hydrolysis.

**2.** A modification of a process as claimed in claim 1 for preparing compounds of formula (I)

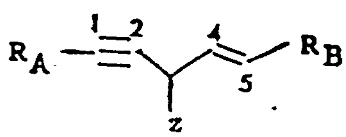

(I)

(wherein,
$R_A$, $R_B$ and Z are as defined in claim 1),
which comprises treating a compound of formula (III)

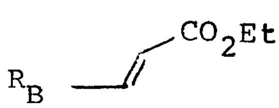

(III)

(wherein $R_B$ is as defined above), with a tert-butyl-dimethylsilyl chloride in the presence of a base, reducing the resultant compound of formula (IV)

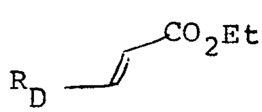

(IV)

(wherein $R_D$ is as defined in claim 1), to the aldehyde or alcohol, optionally converting the resultant alcohol to an allylic halide of formula (V)

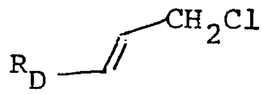
(V)

(wherein $R_D$ is as defined above) reacting said allylic halide or said aldehyde with a compound of formula (VI)

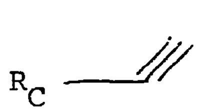
(VI)

(wherein $R_C$ is as defined in claim 1) to form a compound of formula (II) (as defined in claim 1) and subjecting said compound of formula (II) to the alkylammonium fluoride or acid mediated hydrolysis.

3. A modification of the process as claimed in claim 2 wherein the compound of formula (VI) is prepared by the conversion of a compound of formula (VII)

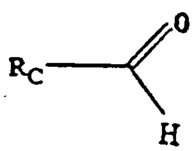
(VII)

(wherein $R_C$ is as defined in claim 1) via a compound of formula (VIII)

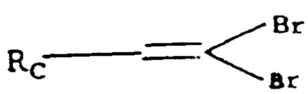
(VIII)

4. A process as claimed in either of claims 1 and 2 wherein the compound of formula (I) produced is 1,5-bis]3'4',3",4"-tetrahydroxyphenyl]pent-4-en-1-yne.

5. A process as claimed in either of claims 1 and 2 wherein the compound of formula (I) produced is 1,5-bis[3;'4',3",4"-tetrahydroxyphenyl]pent-4-en-1-yn-3-ol.

6. A process for preparing compounds of formula (IX)

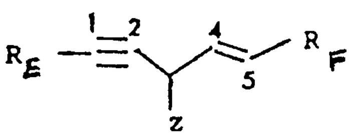
(IX)

(wherein,
$R_E$ represents an unsubstituted phenyl group or a phenyl group substituted by up to 5 groups selected from glucosyloxy, hydroxy, alkoxy, alkylenedioxy, halide and acyloxy groups;

$R_F$ represents an unsubstituted phenyl group or a phenyl group substituted by up to 5 groups selected from glucosyloxy, hydroxy, alkoxy, alkylenedioxy, halide and acyloxy groups;
at least one of the groups $R_E$ and $R_F$ carrying at least one glucosyloxy group; and
Z is as defined in claim 1);
which comprises the glucosidation of a compound of Formula (I) or of Formula (III) in a consecutive step to its preparation by the process as hereinbefore described wherein the Formulae (I) and (II) are as hereinbefore described defined.

**7.** A process as claimed in claim 6 wherein the compound of formula (I) is treated with tetra-acylα-glucosyl bromide in the presence of a Koenigs-Knorr catalyst selected from $Ag_2o$ , $Ag_2CO_3$, $CdCO_3$ or HgCN ,followed by base hydrolysis.

**8.** A process as claimed in claim 6 wherein the compound of formula (I) is treated with a peracyl-glucosyl bromide in the presence of a Koenigs-Knorr catalyst (as defined in claim 7) followed by base hydrolysis.

**9.** A process as claimed in claim 6 wherein the compound of formula (I) is treated with 2,3,4,6-tetra-O-benzyl-α-D-glucopyranosyl fluoride and tetrafluorosilane to provide a mixture of anomeric products.

**10.** A process as claimed in claim 6 wherein the compound of formula (IX) formed is 1,5-bis[3',4',3", 4"-tetrahydroxyphenyl]pent-4-en-1-ynyl-4,4"-di[β-D-glucopyranoside].

## Revendications

**1.** Un procédé de préparation de composés de formule (I):

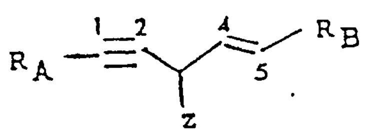

(I)

dans laquelle:
$R_A$ représente un groupe phényle non substitué ou un groupe phényle substitué par jusqu'à 5 groupes choisis parmi les groupes hydroxy, alkoxy, alkylènedioxy, halogénure et acyloxy;
$R_B$ représente un groupe phényle non substitué ou un groupe phényle substitué par jusqu'à 5 groupes choisis parmi les groupes hydroxy, alkoxy, alkylènedioxy, halogénure et acyloxy; .
au moins l'un des groupes $R_A$ et $R_B$ portant au moins un substituant hydroxy; et
z représente un atome d'hydrogène ou un groupe hydroxy;
qui consiste à soumettre un composé de formule (II):

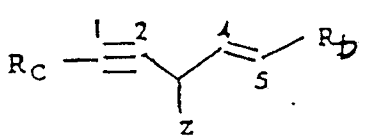

(II)

dans laquelle:
$R_C$ représente un groupe phényle non substitué ou un groupe phényle substitué par jusqu'à 5 groupes choisis parmi les groupes tert-butyldiméthylsilyloxy, alkoxy, alkylènedioxy, halogénure et acyloxy;
$R_D$ représente un groupe phényle non substitué ou un groupe phényle substitué par jusqu'à 5 groupes choisis parmi les groupes tert-butyldiméthylsilyloxy, alkoxy, alkylènedioxy, halogénure et acyloxy;
au moins l'un des groupes $R_C$ et $R_D$ portant au moins un substituant tert-butyldiméthylsilyloxy; et
Z est tel que défini ci-dessus;
à un fluorure d'alkylammonium ou à une acidolyse.

**2.** Une variante d'un procédé selon la revendication 1, pour préparer des composés de formule (I):

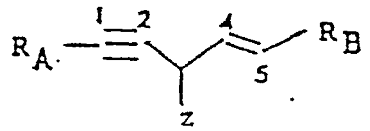

(I)

dans laquelle $R_A$, $R_B$ et Z sont tels que définis dans la revendication 1, qui consiste à traiter un composé de formule (III):

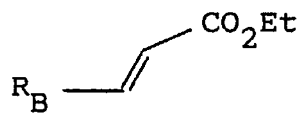

(III)

dans laquelle $R_B$ est tel que défini ci-dessus, avec un chlorure de tert-butyldiméthylsilyle en présence d'une base, à réduire le composé obtenu de formule (IV):

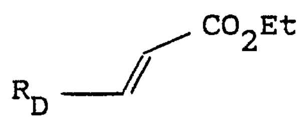

(IV)

dans laquelle $R_D$ est tel que défini dans la revendication 1, en l'aldéhyde ou l'alcool, facultativement à convertir l'alcool obtenu en un halogénure allylique de formule (V):

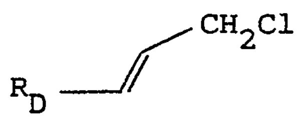

(V)

dans laquelle $R_D$ est tel que défini ci-dessus, à faire réagir ledit halogénure allylique ou ledit aldéhyde avec un composé de formule (VI)

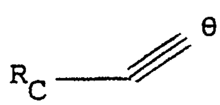

(VI)

dans laquelle $R_C$ est tel que défini dans la revendication 1, pour former le composé de formule (II) (tel que défini dans la revendication 1), et à soumettre ledit composé de formule (II) au fluorure d'alkylammonium ou à l'acidolyse.

3. Une variante du procédé selon la revendication 2, dans laquelle le composé de formule (VI) est préparé par conversion d'un composé de formule (VII):

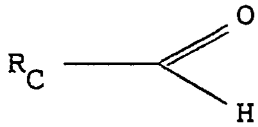

(VII)

dans laquelle $R_C$ est tel que défini dans la revendication 1, par l'intermédiaire d'un composé de formule (VIII):

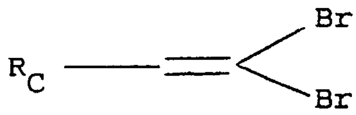

(VIII)

4. Un procédé selon l'une ou l'autre des revendications 1 et 2, dans lequel le composé de formule (I) tel que produit est le l,5-bis[3',4',3",4"-tétrahydroxyphényl]pent-4-èn-1-yne.

5. Un procédé selon l'une ou l'autre des revendications 1 et 2, dans lequel le composé de formule (I) tel que produit est le 1,5-bis[3',4',3",4"-tétrahydroxyphényl]pent-4-èn-l-yn-3-ol.

6. Un procédé de préparation de composés de formule (IX):

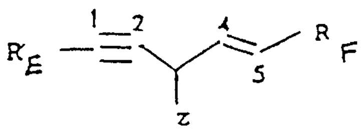

(IX)

dans laquelle:
$R_E$ représente un groupe phényle non substitué ou un groupe phényle substitué par jusqu' à 5 groupes choisis parmi les groupes glucosyloxy, hydroxy, alkoxy, alkylènedioxy, halogénure et acyloxy;
$R_F$ représente un groupe phényle non substitué ou un groupe phényle substitué par jusqu'à 5 groupes choisis parmi les groupes glucosyloxy, hydroxy, alkoxy, alkylènedioxy, halogénure et acyloxy;
au moins l'un des groupes $R_E$ et $R_F$ portant au moins un groupe glucosyloxy; et
Z est tel que défini dans la revendication 1;
qui comprend la glucosidation d'un composé de formule (I) ou de formule (II) dans une étape ultérieure à sa préparation, par le procédé tel que décrit ci-dessus, dans lequel les formules (I) et (II) sont telles que définies ci-dessus.

7. Un procédé selon la revendication 6, dans lequel le composé de formule (I) est traité par du bromure de tétra-acyl-α-glucosyle en présence d'un catalyseur de Koenigs-Knorr choisi parmi $Ag_2O$, $Ag_2CO_3$, $CdCO_3$ et HgCN, opération suivie d'une hydrolyse basique.

8. Un procédé selon la revendication 6, dans lequel le composé de formule (I) est traité par un bromure de peracylglucosyle en présence d'un catalyseur de Koenigs-Knorr (tel que défini dans la revendication 7), opération suivie d'une hydrolyse basique.

9. Un procédé selon la revendication 6, dans lequel le composé de formule (I) est traité avec du fluorure de 2,3,4,6-tétra-O-benzyl-α-D-glucopyrannosyle et du tétrafluorosilane pour donner un mélange de produits anomères.

10. Un procédé selon la revendication 6, dans lequel le composé de formule (IX) tel que formé est le 1,5-bis-[3' ,4' ,3",4"-tétrahydroxyphényl]pent-4-èn-1-ynyl-4,4"-di[ß-D-glucopyrannoside].

## Ansprüche

1. Verfahren zur Herstellung von Verbindungen der Formel (I)

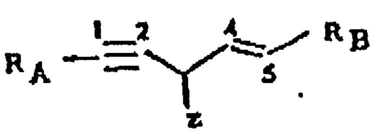

(I)

(in der $R_A$ eine unsubstituierte Phenylgruppe oder eine Phenylgruppe, die durch bis zu 5 Gruppen substituiert ist, ausgewählt aus Hydroxy-` Alkoxy-, Alkylendioxy-, Halogenid- und Acyloxygruppen, bedeutet;
$R_B$ eine unsubstituierte Phenylgruppe oder eine Phenylgruppe, die durch die zu 5 Gruppen substituiert ist, ausgewählt aus Hydroxy-, Alkoxy-, Alkylendioxy-, Halogenid- und Acyloxygruppen, bedeutet;
mindestens eine der Gruppen $R_A$ und $R_B$ mindestens einen Hydroxysubstituienten enthält; und Z ein Wasserstoffatom oder eine Hydroxygruppe bedeutet); wobei man eine Verbindung der Formal (II)

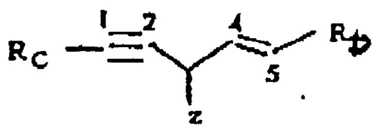

(II)

(in der $R_C$ eine unsubtituierte Phenylgruppe oder eine Penylgruppe, die substituiert ist durch bis zu 5 Gruppen, ausgewählt aus tert.Butyldimethylsilyloxy-, Alkoxy-, Alkylendioxy-, Halogenid-und Acyloxygruppen, bedeutet;
$R_D$ eine unsubstituierte Phenylgruppe oder eine Phenylgruppe, die substituiert ist durch bis zu 5 Gruppen, ausgewählt aus tert.Butyldimethylsilyloxy`-, Alkoxy-, Alkylendioxy-, Halogenid-und Acyloxygruppen, bedeutet;
mindestens eine der Gruppen $R_C$ und $R_D$ mindestens einen tert.Butyldimethylsilyloxy-Substituenten enthält; und
Z wie oben definiert ist),
der Hydrolyse mit Hilfe von Alkylammonium-fluorid oder Säure unterwirft.

2. Modifizierung des Verfahrens nach Anspruch 1 zur Herstellung von Verbindungen der Formel (I)

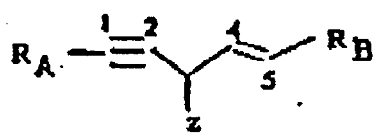

(I)

(in der $R_A$ , $R_A$ und Z wie in Anspruch 1 definiert sind), umfassend die Behandlung einer Verbindung der Formel (III)

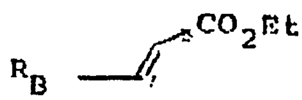

(III)

(wobei $R_B$ wie oben definiert ist), mit einem tert.Butyldimethylsilylchlorid in Gegenwart einer Base, Reduktion der erhaltenen Verbindung der Formel (IV)

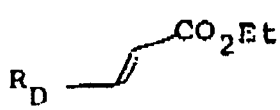

(IV)

(wobei $R_D$ wie in Anspruch 1 definiert ist), zu dem Aldehyd oder Alkohol, gegebenenfalls Umwandlung des erhaltenen Alkohols in ein Allylhalogenid dem Formel (V)

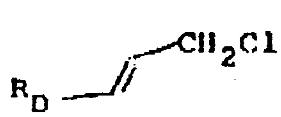

(V)

(wobei $R_D$ wie oben definiert ist), Umsetzung dieses Allylhalogenids oder des Aldehyds mit einer Verbindung der Formel (VI)

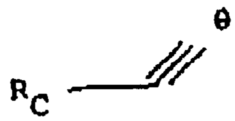

(VI)

(wobei $R_C$ wie in Anspruch 1 definiert ist) unter Bildung einer Verbindung der Formel (II) (wie in Anspruch 1 definiert) und Hydrolyse dieser Verbindung der Formel (II) mit Hilfe von Alkylammoniumfluorid oder Säure.

3. Modifizierung des Verfahrens nach Anspruch 2, wobei die Verbindung der Formel (VI) hergestellt wird durch Umwandlung einer Verbindung der Formel (VII)

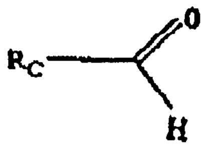

(VII)

(in der $R_C$ wie in Anspruch 1 definiert ist) Über eine Verbindung der Formel (VIII)

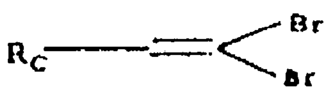

(VIII)

4. verfahren nach einem der Ansprüche 1 oder 2, wobei die hergestellte Verbindung der Formel (I) 1,5-Bis[3',4',3",4"-tetrahydroxyphenyl]pent-4-en-in-3-ol ist.

5. verfahren nach einem der Ansprüche 1 oder 2, wobei die hergestellte Verbindung der Formel (I) 1,5-Bis[3',4',3",4"-tetrahydroxyphenyl]pent-4-en-l-in-3-ol ist.

6. verfahren zur Herstellung von Verbindungen der Formel (IX)

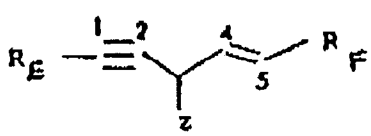

(IX)

(wobei $R_E$ eine unsubstituierte Phenylgruppe oder eine Phenyl-gruppe, die substituiert ist durch bis zu 5 Gruppen, ausgewählt aus Glucosyloxy-, Hydroxy-, Alkoxy-, Alkylendioxy-, Halogenid-und Acyloxygruppen, bedeutet;
$R_F$ eine unsubstituierte Phenylgruppe oder eine Phenylgruppe, die substituiert ist durch bis zu 5 Gruppen, ausgewählt aus Glucosyloxy-, Hydroxy-, Alkoxy-, Alkylendioxy-, Halogenid- und Acyloxygruppen, bedeutet;
mindestens eine der Gruppen $R_E$ und $R_F$ mindestens eine Glucosyloxygruppe enthält; und
Z wie in Anspruch 1 definiert ist);
umfassend die Glucosidierung einer Verbindung der Formel (I) oder der Formel (II) in einer Stufe nach der Herstellung nach dem oben beschriebenen Verfahren, wobei die Formeln (I) und (II) wie oben definiert sind.

7. Verfahren nach Anspruch 6, wobei die Verbindung der Formel (I) behandelt wird mit Tetraacyl-α-glucosylbromid in Gegenwart eines Koenigs-Knorr-Katalysators, ausgewählt aus $Ag_2O$, $Ag_2CO_3$, $CdCO_3$ und HgCN, und anschließende Basenhydrolyse.

**8.** Verfahren nach Anspruch 6, wobei die Verbindung der Formel (I) behandelt wird mit einem Peracylglucosylbromid in Gegenwart eines Koenigs-Knorr-Katalysators (wie in Anspruch 7 definiert) und anschließende Basehydrolyse.

**9.** verfahren nach Anspruch 6, wobei die Verbindung der Formel (I) behandelt wird mit 2,3,4,6-Tetra-O-benzyl-α-D-glucopyranosyl-fluorid und Tetrafluorsilan unter Bildung eines Gemisches anomerer Produkte.

**10.** verfahren nach Anspruch 6, wobei die entstandene Verbindung der Formel (IX) 1,5-Bis[3',4',3",4"-tetrahydroxyphenyl]-pent-en-l-inyl-4,4"-di[β-D-glucopyranosid] ist.